# EUROPÄISCHE PATENTSCHRIFT

(11) **EP 0 852 723 B1**
(45) Veröffentlichungstag und Bekanntmachung des Hinweises auf die Patenterteilung: **03.07.2002**
(21) Anmeldenummer: 96944561.8
(22) Anmeldetag: 18.09.1996
(51) Int. Cl.: G01N 33/00, G01N 33/497

(54) **BIOSENSORSYSTEM ZUR MESSUNG EINER ODER MEHRERER, INSBESONDERE ORGANISCHER, DURCH PFLANZENSCHÄDIGUNGEN VERURSACHTEN SPURENKOMPONENTEN IN LUFT**
BIOSENSOR SYSTEM FOR MEASURING ONE OR MORE TRACE COMPONENTS, IN PARTICULAR ORGANIC COMPONENTS, IN AIR RESULTING FROM CROP DAMAGE
SYSTEME DE BIODETECTEUR POUR MESURER LA PRESENCE D'AU MOINS UN ELEMENT DE TRACE DANS L'AIR, NOTAMMENT UN ELEMENT ORGANIQUE, RESULTANT DE DEGATS PROVOQUES SUR DES VEGETAUX

(30) Priorität: 29.09.1995 DE 19536389
(43) Veröffentlichungstag der Anmeldung: 15.07.1998
(73) Patentinhaber: FORSCHUNGSZENTRUM JÜLICH GMBH, 52425 Jülich (DE)
(72) Erfinder: SCHÖNING, Michael, Josef, D-52428 Jülich (DE); SCHÜTZ, Stefan, D-35392 Gie en (DE); RIEMER, Armin, D-41516 Grevenbroich (DE); THUST, Marion, D-50939 Köln (DE); WEISSBECKER, Bernhard, D-63534 Gründau (DE); KOHL, Claus-Dieter, D-35396 Gie en (DE); HUMMEL, Hans, E., D-35394 Gie en (DE); KORDOS, Peter, D-52428 Jülich (DE); LÜTH, Hans, D-52076 Aachen (DE)
(86) Internationale Anmeldenummer: DE9601800
(87) Internationale Veröffentlichungsnummer: WO9712219

(56) Entgegenhaltungen:
- EP-A- 0 258 951
- DE-A- 3 906 004
- DE-U- 9 209 302
- IEEE TRANSACTIONS ON COMPONENTS,HYBRIDS,AND MANUFACTURING TECHNOLOGY, Bd. CHMT-10, Nr. 2, Juni 1987, NEW YORK US, Seiten 267-273, XP002027580 MASAYOSHI KANEYASU, ET AL.: "Smell Identification Using a Thick-Film Hybrid Gas Sensor"

## Beschreibung

Die Erfindung betrifft ein Biosensorsystem zur Messung einer oder mehrerer Spurenkomponenten pflanzlichen oder mikroorganismischen Ursprungs in Luft, gemäß dem Oberbegriff des Anspruchs 1.

Eines der Hauptprobleme im integrierten Pflanzenschutz ist die Bestimmung des optimalen Applikationszeitpunktes des Insektizids, der zu einer beträchtlichen Verminderung des Verbrauchs führt und einer schnellen Ausbildung von Resistenzen bei Schadinsekten entgegenwirkt.

Aufgrund der großen ökonomischen und ökologischen Bedeutung dieses Problems für die Pflanzenproduktion wurden verschiedene Methoden entwickelt, um einen günstigen Behandlungszeitpunkt festzulegen (R.L. Metcalf et al., Introduction of insect pest management, John Wiley & Sons, New York 1975).

Diese Methoden basieren entweder auf regelmäßigen, zeitraubenden Begehungen der Felder mit Erfassung der vorhandenen Populationsdichte über das Abzählen von Schadinsekten oder auf komplizierten Computersimulationsmodellen der Schädlings-Populationsentwicklung, die jedoch nur lokal und auf einzelne Sorten anwendbar sind.

Eine universellere Methode zur Bestimmung des Applikationszeitpunktes besteht in der Messung des Ausmaßes der verletzungsbedingten Duftstoffemission von Pflanzen. Die Messung dieser Emissionen kann sowohl mittels konventioneller Spurenanalytik wie z.B. die Gas-Chromatographie in Kombination mit der Massenspektroskopie (GC-MS) als auch in jüngster Zeit mittels der Methode eines Elektroantennogramms (EAG) erfolgen.

Bei der in der Spurenanalytik häufig verwendeten GC-MS-Methode (J. Arey et al., Terpenes emitted from agricultural species found in Californias central valley, J. Geophs. Res. 96 (1991), 9329-36) stehen der hohen Zuverlässigkeit und Empfindlichkeit einerseits, hoher Personal- und Kostenaufwand sowie zeitliche und räumliche Schwerfälligkeit aufgrund mangelnder Miniaturisierbarkeit entgegen.

Im Gegensatz dazu ermöglicht die EAG-Methode die schnelle und hochsensitive Detektion von Emissionen im Bereich unterhalb von 1 ppm, wie bereits von S. Schütz et al., Biosensor for plant damage by insects, Med. Fa. Lanbouww., Uinversität Gent, Belgien, 59/2b (1994) berichtet wurde.

Bei dieser Methode wird ein Insekt fest eingespannt und der Spannungsabfall an der Insektenantenne durch Anbringen einer Mikroelektrode am freien Ende der Insektenantenne sowie einer weiteren Mikroelektrode nahe dem mit dem Kopf verbundenen Ende der Insektenantenne gemessen. Dabei ist mit diesen beiden Elektroden über Zufuhrdrähte eine spezielle Signalverarbeitung verbunden. Gemessen wird dabei der zeitliche Verlauf der Depolarisationsspannung in der Insektenantenne, wenn Spurenkomponente enthaltende Luft an der Antenne vorbeiströmt.

Nachteilig bei diesem Biosensorsystem wirkt sich die elektrische und mechanische Störanfälligkeit des Meßaufbaus, sowie die unzureichende Lebensdauer der biosensitiven Komponente, aufgrund der ungeeigneten Adaption der Insektenantenne an die Ableitelektrode aus. Dies führt zu einer geringen Flexibilität im Betrieb und bei der Handhabung. Es sind zudem Mikromanipulatoren zur stabilen Kopplung der jeweiligen Elektroden an die Antenne notwendig. Schließlich handelt es sich bei den Mikroelektroden um mechanisch sehr empfindliche, aus z.B. Glas mit einem Durchmesser von beispielsweise 1 µm hergestellte Elektroden.

Es ist deshalb Aufgabe der Erfindung, einen Biosensor zu schaffen, sowie ein geeignetes Herstellungs- und Präparationsverfahren bereitzustellen, bei dem eine hohe Sensitivität und Selektivität sowie geringe Störanfälligkeit und portabler Einsatz für Feldmessungen zur Detektion von verletzungsinduzierten, pflanzlichen Emissionen, wie z.B. Terpene oder Blattalkohole, erreicht wird. Die Messung dieser Emission soll eine gezielte Abschätzung von Art und Stärke der pflanzlichen Verletzung und dadurch eine präzise Bewertung des optimalen Applikationszeitpunktes von Pestiziden ermöglichen.

Die Aufgabe wird gelöst durch einen Biosensor gemäß der Gesamtheit der Merkmale nach Anspruch 1. Weitere zweckmäßige oder vorteilhafte Ausführungsformen finden sich in den auf den Anspruch 1 rückbezogenen Unteransprüchen.

Das entwickelte Biosensorsystem besteht aus einer Meßluftzuführung, einer Biokomponente, einer Halbleiterkomponente, einer Probenzellenanordnung sowie einer Signal- und Meßwertverarbeitung. Die Meßluftzuführung ermöglicht die definierte Probennahme für die Biokomponente. Die Biokomponente besteht aus einem intakten Chemorezeptor, z.B. der Insektenantenne eines Kartoffelkäfers. Dabei kann der Chemorezeptor gleichstrommäßig oder auch wechselstrommäßig (z.B kapazitiv oder induktiv) mit der Halbleiterkomponente verbunden sein.

Der Erkennungsmechanismus erfolgt nach Durchtritt durch die Poren der schützenden Chitin-Cuticula in die Rezeptorlymphe dieser Insektenantenne durch hochspezifische (Pheromene Binding Protein = PBP) oder gruppenspezifische (General Odorant Binding Protein = GOBP) Proteine, die ihrerseits an die membranbeständigen Rezeptoren oder Dendriten binden und dadurch die Depolarisation der Neuronenmembran bewirken. Diese Depolarisationen werden mit hochohmigen Halbleiter-Verstärkern, z.B. Feldeffekttransistoren, unmittelbar meßbar gemacht. Mittels einer geeigneten Signal- und Meßwertverarbeitung ist die Menge der auslösenden Substanz (Spurenkomponente in der Luft) über mehrere Größenordnungen quantifizierbar.

Die erforderliche, hohe Sensitivität und Selektivität des Biosensorsystems wird durch die geeignete Auswahl der Biokomponente, - Art, Zucht und Präparation der verwendeten Insektenantenne, z.B. aus der Familie der Blattkäfer -, in Abhängigkeit des Nachweises der erwünschten Spurenkomponente in der zu untersuchenden Luft erreicht oder sogar weiter optimiert. Aufgrund der Miniaturisierbarkeit der Schnittstelle zwischen intaktem Chemorezeptor und Halbleiterkomponente in Form einer Verbindungseinheit wird eine höhere mechanische Störsicherheit sowie eine deutliche Reduzierung der elektrischen Störanfälligkeit erzielt. Die Miniaturisierung ermöglicht weiterhin die Konstruktion eines kompakten, robusten und portablen Biosensorsystems für den Einsatz in Feldmessungen.

Die Erfindung ist im weiteren an Hand von Figuren und Ausführungsbeispiel näher erläutert. Es zeigen:
- Fig. 1:: Biosensorsystem;
- Fig. 2:: Vergrößerte Darstellung des Systems nach Figur 1 im Bereich des Biosensors;

### Ausführungsbeispiel

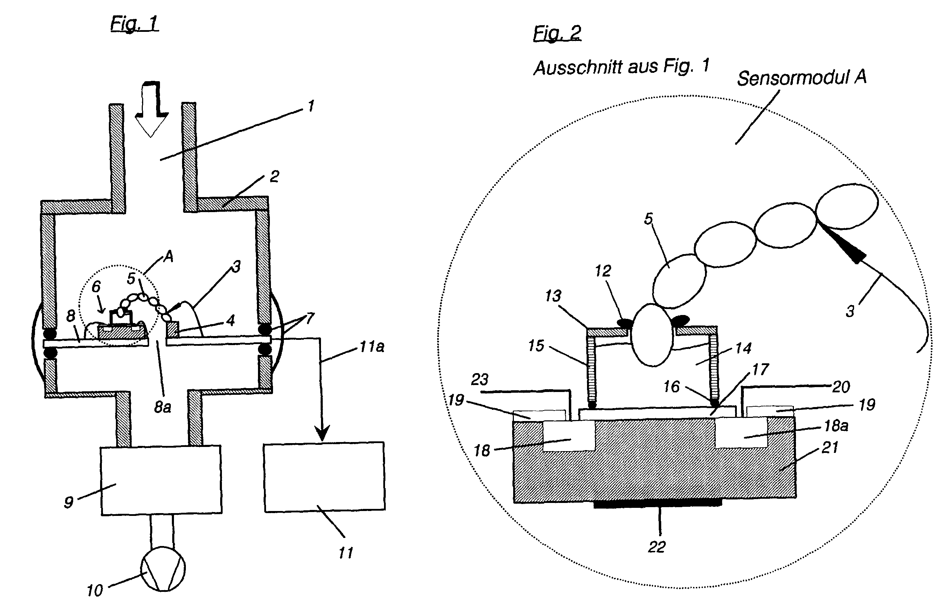

In Figur 1 ist das entwickelte Biosensorsystem mit intaktem Chemorezeptor in Form der Antenne eines Kartoffelkäfers dargestellt. Ein solches System kann Einsatz finden z.B. im Bereich der Schädigung von Kartoffelpflanzen durch Kartoffelkäfer.

Die Meßluftzuführung 1 aus einem festen oder mittels Kugelschliff beweglichen Rohr aus z.B. Glas, Teflon oder Edelstahl mit einem Durchmesser im Bereich von bis zu 100 mm, insbesondere zwischen 5 und 15 mm, mündet in eine Probenzellenanordnung 2 aus z.B. Glas, Teflon oder Edelstahl. Die Probenzellenanordnung ermöglicht einen einfachen und schnellen Wechsel bzw. Austausch des Sensormoduls 5,6 und gewährt die für den portablen Einsatz erforderliche, mechanische Stabilität.

Über geeignete mikromechanische Arretier- bzw. Haltevorrichtungen 7 wird das Sensormodul 5,6 und folglich die Biokomponente stabil und geschützt positioniert. Eine weitere Erhöhung der elektrischen Störsicherheit kann z.B. durch das Einbringen des Sensormoduls in einen miniaturisierten, auf einem stabilen elektrischen Potential befindlichen Faradaykäfig erzielt werden.

Das Sensormodul 5,6 wird mit einem festen Träger 8, z.B. mit einem mit einer Öffnung 8a für die durchströmende Luft zur Beprobung der Biokomponente versehenen Leiterplattensubstrat 8, verbunden. Zur weiteren mechanischen Stabilisierung der Antenne 5 ist jenseits der Öffnung 8a eine elektrisch isolierende Stütze 4 vorgesehen. Beispielsweise über ein als Pulsationsdämpfer wirkendes Gefäß 9 kann die die zu detektierende Spurenkomponente enthaltende Meßluft von einer Pumpe 10 mit einem der Probenzellenanordnung angemessenen Durchsatz von beispielsweise bis zu 5 l/min, vorzugsweise im Bereich 0,5 - 1,5 l/min, abgesaugt werden.

Die vom Sensormodul 5,6 erhaltenen Meßsignale werden über vorzugsweise abgeschirmte Zuleitungen 11a an eine Signal- und Meßverarbeitungseinheit 11 weitergeleitet. Das resultierende Signal korreliert dabei direkt mit der Intensität der pflanzlichen Emission, und damit im Ergebnis mit der Art und Stärke der Schädigung der Pflanzen.

Der in Figur 2 dargestellte Biosensor 5,6 setzt sich aus einer Halbleiterkomponente, vorzugsweise einem Feldeffekttransistor (FET, z.B. einem MOSFET) einer Insektenantenne 5, z.B. dem intakten Chemorezeptor 5 eines Kartoffelkäfers, sowie einer Verbindungseinheit, die den elektrischen Kontakt zwischen der Antenne und dem FET bewirkt, zusammen.

Der intakte Chemorezeptor 5 ist am einen Ende über eine Ableiterelektrode 3 aus z.B. Glas, Metall, vorzugsweise Ag, Pt oder Ag/AgCl kontaktiert. Am anderen Ende ist sie über einen Flüssig- oder Festkontakt 14, vorzugsweise eine Elektrolytlösung 14, direkt elektrisch in Verbindung mit der Halbleiterkomponente gebracht. Es kann außerdem an diesem Ende eine zusätzliche Referenzelektrode, z.B. aus Ag/AgCl, vorgesehenen sein um auf diese Weise mit der Ableitlelektrode 3 am anderen Ende den unmittelbaren Spannungsabfall an der Insektenantenne 5 zu bestimmen.

Die Antenne (intakter Chemorezeptor) 5 wird mechanisch und elektrisch stabil über eine Antennenhaltevorrichtung, beispielsweise bestehend aus einer mit einem viskosen Dichtmaterial 12 abgedichteten Sockelplatte 13, durch die die Antenne zum Flüssig- und Festkontakt durchgeführt werden kann, gehalten. Dabei ist es vorteilhaft möglichst wenig Einzelglieder der Insektenantenne in den Elektrolyten zu tauchen, sodaß ein möglichst langer Bereich der Antenne 5 zur Detektierung der erwünschten Spurenkomponente in die Antenne 5 vorbeiströmenden Meßluft eingesetzt wird. Die Sockelplatte 13 ist direkt und mechanisch stabil mit der Wandung 15 verbunden, die ihrerseits über eine Dichtung 16, z.B. O-Ring gedichtet, direkt mit der Halbleiterkomponente verbunden ist.

Die Halbleiterkomponente, vorzugsweise ein FET-Bauelement setzt sich aus einem Grundsubstrat 21 aus z.B. n- bzw. p-dotiertes Silicium zusammen. Abhängig von der Dotierung dieses Grundmaterials weisen die taschenförmige, zur Ausbildung von Source und Drain vorgesehenen Substratbereiche 18 und 18a eine entgegengesetzte Dotierung auf. Diese Substratbereiche 18 und 18a sind über eine vorzugsweise metallische Kontaktierung 20 sowie einen rückseitigen Kontakt 22 (z.B. Ti/Al, Ti/Pd/Au o.ä. leitende Materialien) mit einem festen Träger, z.B. mit dem Leiterplattensubstrat 8, elektrisch passiviert und verkapselt, verbunden. Die Metallisierung 20 kann durch eine Isolatorschicht bzw. -schichtfolge 19 aus SiO₂ oder SiO₂/Si₃N₄ oder SiO₂/Al₂O₃ o.ä. vom Grundsubstrat 22 isoliert, schichtförmig aufgebracht sein. Anstelle der üblichen Gateelektrode beim FET ist bei dieser Anordnung die Kontaktierung des intakten Chemorezeptors mittels der Elektrolytlösung 14, die gleichzeitig die biologische Nährlösung für die Antenne realisiert. Alternativ zum Elektrolyten wären andere Flüssig- oder auch Festkontakte vorstellbar. In der in Figur 1 und Figur 2 dargestellten Anordnung ist ein flüssigkeitsresistiver Gateisolator 17, z.B. aus SiO₂ oder SiO₂/Si₃N₄ oder SiO₂/Al₂O₃ mit einer Schichtdicke von beispielsweise einigen nm, eingesetzt.

Die direkte Kontaktierung des intakten Chemorezeptors mit der Halbleiterkomponente ermöglicht eine deutliche Verringerung der Störanfälligkeit des Biosensorsystems aufgrund der direkten hochohmigen, rauscharmen Signalverstärkung durch den miniaturisierten, als Signalwandler eingesetzten FET. Dieser direkte Kontakt ermöglicht eine schnelle und schonende, d.h. geringfügig in das Hämolymphesystem der Antenne eingreifende, biologische Präparation, die einerseits eine zuverlässige Signalübertragung zum FET leistet und die andererseits den mit Autolysereaktionen und Ionenverteilungsungleichgewichten verbundenen Eingriff in das biologische Funktionssystem so gering wie möglich hält. Soweit die in den Sensor präparierte Antenne verbraucht ist, kann auf relativ einfache Weise eine neue Antenne an der Stelle der alten präpariert werden. Auf diese Weise ist der übrige Aufbau oftmals wiederverwendbar.

Durch die Entwicklung eines vergleichsweise billigen und mobilen Biosensorsystems mit intakten Chemorezeptoren kann die Messung von Pflanzenschäden auf der Basis von biologischen Emissionen in einem landwirtschaftlich sinnvollen Skalenbereich realisiert werden, da üblicherweise eingesetzte Methoden zur Erfassung von Pflanzenschäden nachteilig sind, aufgrund der Tatsache einer zu kleinräumigen (Begehung von Bonitur) oder zu großräumigen (Infrarotaufnahmen per Satellit oder Flugzeug) Erfassungskontrolle.

## Patentansprüche

1. Biosensor mit einem zur Detektierung einer oder mehrerer Spurenkomponenten in Luft vorgesehenem Chemorezeptor(5), wobei dieser als intakter Chemorezeptor, insbesondere Insektenantenne(5) ausgebildet ist, **dadurch gekennzeichnet, daß** der Chemorezeptor(5) mittelbar über einen Elektrolyten(14) oder unmittelbar mit einer Halbleiterkomponente verbunden ist.

2. Biosensor nach Anspruch 1, **dadurch gekennzeichnet, daß** eine geringe Zahl an Antennenkettenglieder, insbesondere ein einziges Antennenkettenglied, am einen Ende des Chemorezeptors(5) direkt oder über dem Elektrolyten(14) mit der Halbleiterkomponente verbunden ist.

3. Biosensor nach Anspruch 1 oder 2, **dadurch gekennzeichnet, daß** als Elektrolyt(14) ein Fest- oder Flüssigelektrolyt(14) vorgesehen ist.

4. Biosensor nach einem der vorhergehenden Ansprüche, **dadurch gekennzeichnet, daß** als Halbleiterkomponente eine auf dem Feldeffekt beruhende Halbleiter-Isolatorstruktur, insbesondere ein Permeable Base Transistor (PBT) oder ein FET, vorgesehen ist.

5. Biosensor nach Anspruch 4, **dadurch gekennzeichnet, daß** der Chemorezeptor(5) mit dieser Halbleiter-Isolatorstruktur so verbunden ist, daß er die Gate-Elektrode des PBTs bzw. FETs bildet.

6. Biosensor nach einem der vorhergehenden Ansprüche, **dadurch gekennzeichnet, daß** der Chemorezeptor(5) an mehreren Stellen, insbesondere im Falle einer Insektenantenne an mehreren Stellen dieser oder deren Sinneshärchen, mit der Meßelektronik kontaktiert ist und eine weitere Stelle mit der Halbleiterkomponente verbunden ist.

## Claims

1. Biosensor with a chemoreceptor (5) provided for detecting one or more trace components in air, whereby this is designed as an intact chemoreceptor, particularly an insect antenna (5), **characterised in that** the chemoreceptor (5) is linked indirectly, via an electrolyte (14), or directly to a semiconductor component.

2. Biosensor according to Claim 1, **characterised in that** a smaller number of antenna chain members, particularly a single antenna chain member, at one end of the chemoreceptor (5) is linked directly or via an electrolyte (14) to the semiconductor component.

3. Biosensor according to Claim 1 or Claim 2, **characterised in that**, as electrolyte (14), a solid or liquid electrolyte (14) is provided.

4. Biosensor according to one of the previous claims, **characterised in that**, as semiconductor component, a semiconductor-insulator structure relying on the field effect, particularly a permeable base transistor (PBT) or an FET, is provided.

5. Biosensor according to Claim 4, **characterised in that** the chemoreceptor (5) is linked to this semiconductor-insulator structure such that it comprises the gate electrode of the PBT or FET.

6. Biosensor according to one of the previous claims, **characterised in that** the chemoreceptor (5) is linked to the measuring electronics at a plurality of sites, particularly in the case of an insect antenna, at a plurality of sites on same or its sensory hairs and, at a further site, to the semiconductor component.

## Revendications

1. Biodétecteur avec un chimiorécepteur (5) prévu pour la détection d'un ou de plusieurs éléments de trace dans l'air, celui-ci étant conçu comme un chimiorécepteur intact, en particulier une antenne d'insecte (5), **caractérisé en ce que** le chimiorécepteur (5) est relié indirectement par un électrolyte (14) ou directement à un composant semi-conducteur.

2. Biodétecteur selon la revendication 1, **caractérisé en ce qu'**un faible nombre de maillons d'antenne, en particulier un maillon d'antenne unique, est relié sur une extrémité du chimiorécepteur (5) directement ou par l'intermédiaire de l'électrolyte (14) au composant semi-conducteur.

3. Biodétecteur selon la revendication 1 ou 2, **caractérisé en ce qu'**il est prévu comme électrolyte un électrolyte solide ou liquide (14).

4. Biodétecteur selon l'une quelconque des revendications précédentes, **caractérisé en ce qu'**il est prévu comme composant semi-conducteur une structure d'isolateur à semi-conducteurs basé sur l'effet de champ, en particulier un Permeable Base Transistor (PBT) ou un FET.

5. Biodétecteur selon la revendication 4, **caractérisé en ce que** le chimiorécepteur (5) est relié à cette structure d'isolateur à semi-conducteurs de telle façon qu'il constitue l'électrode de grille du PBT ou FET.

6. Biodétecteur selon l'une quelconque des revendications précédentes, **caractérisé en ce que** le chimiorécepteur (5) est en contact avec l'électronique de mesure en plusieurs endroits, en particulier dans le cas d'une antenne d'insecte en plusieurs endroits de celle-ci ou de ses poils sensoriels et un autre endroit est relié au composant semi-conducteur.
